# EUROPEAN PATENT APPLICATION

(11) **EP 1 306 366 A2**
(43) Date of publication of application: **02.05.2003**
(21) Application number: 03002060.6
(22) Date of filing: 26.11.1998
(51) Int. Cl.: C07C 235/84, C07C 255/56

(54) **New dibenzosuberone derivatives**

(30) Priority: 01.12.1997 HR 970649
(62) Divisional of application: 98957044.5
(71) Applicant: Belupo - Lijekovi I Kozmetika D.O.O., 48000 Koprivnica (HR)
(72) Inventor: Mikotic-Mihun, Zvonimira, Ph. D., 10000 Zagreb (HR); Karminski Zamola, Grace,, 10000 Zagreb (HR); Cepanec, Ivica, Ph. D., Kneginec Gornji, 42204 Turcin (HR); Litvic, Mladen, M. S., 48326 Virje (HR)
(74) Representative: Rupprecht, Kay, Dipl.-Ing.

(57) **Abstract**

The invention referres to the new dibenzosuberone derivatives *N,N'-bis*(3'-dimethylamino-1'-propyl)dibenzosuberone-3,7-*bis*-carboxamide **(1)** and to the process for its preparation *via* 3,7-di-cyano-dibenzosuberone **(3).**

## Description

The invention is referred to the new dibenzosuberone derivative, *N,N*'-*bis*(dimethylamino-1'-propyl)dibenzosuberone-3,7-biscarboxymide hydrochloride (**1**) and the procedures of its preparation. The preparation of bromo derivatives of dibenzosuberone by total synthesis from *p*-bromophenylacetic acid and phtalic anhydride, as well as some other alternative synthetic procedures have also been described [Ebnoether et al., Helv. Chim. Acta, 48 (1965) 1237; Bastian et al., Helv. Chim. Acta, 49 (1966) 214]. Preparation of various monosubstituted dibenzosuberone derivatives have been described as well [Hrnciar, Helv. Chem. Zvesti, XVI 1-2 (1962) 96]. The subject of this invention is *bis*-amide **1**, and procedure for its preparation starting from 3,7-dibromo dibenzosuberone (**2**) *via bis*-nitrile **3** and *bis*-carboxylic acid **4**: 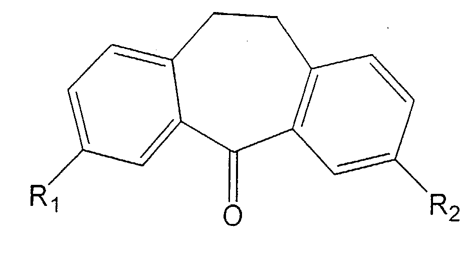
**1**: R₁, R₂= CONHCH₂CH₂CH₂NH(CH₃)₂⁺ Cl⁻
**2**: R₁, R₂= Br
**3**: R₁, R₂= CN
**4**: R₁, R₂= COOH

In the first step 3,7-dibromo dibenzosuberone (**2**) was converted to *bis*-nitrile **3** by reaction with copper(I) cyanide (4 eq.) in quinoline as reaction solvent at 100-230 °C during 1-24 hours. Alternative reaction solvents are pyridine and isomeric lutidines at reflux temperatures. Product 3 was purified with refluxing acetone.

*Bis*-nitrile **3** was converted to *bis*-acid **4** by acid-catalysed hydrolysis. Reaction was performed by heating the bis-nitrile 3 with excess of concentrated hydrochloric acid and acetic acid during 30-50 hours.

Finally, *bis*-amide **1** was prepared by reaction of bis-acid **4** with thionyl chloride followed by reaction with 3-dimethylamino-1-propylamine in lower ethers eg. tetrahydrofurane, or chlorinated solvents eg. dichloromethane as reaction solvents at -5-10 °C during 0.5-24 h. *Bis*-hydrochloride of product **1** was obtained by introduction of dry hydrogenchloride into the solution of base form of compound **1** in lower aliphatic alcohols eg. ethanol at -70- +20 "C, followed by evaporation and vacuum drying.

### Example 1

Preparation of 3,7-dicyano dibenzosuberone (3)

Copper(I) cyanide (6.81 g, 0.076 mol) was added to the solution of 3,7-dibromo dibenzosuberone (2, 6.96 g, 0.019 mol) in 50 ml of quinoline. Reaction mixture was stirred at reflux temperature, ca. 230 °C, during 80 minutes. Then the reaction solvent was evaporated and residue was heated with 150 ml of concentrated hydrochloric acid and extracted with 100 and 2x50 ml of dichloromethane. Organic extracts were collected, dryed over anhydrous sodium sulphate, filtered and evaporated. Crude product was purified with refluxing acetone. 3,7-dicyano dibenzosuberone (2.64 g) was obtained as white powder, mp. 226.5-227.5 °C.
IR (KBr) ν: 3460, 3100, 2970, 2940, 2880, 2260, 1650, 1610, 1600, 1500, 1455, 1435, 1420, 1370, 1320, 1290, 1245, 1200, 1190, 1150, 1140, 1105, 1030, 970, 940, 865, 840, 815, 805, 650, 615 cm⁻¹.
¹H-NMR (CDCl₃) δ: 3.31 (s, 4H, benzyls), 7.41 (d, 2H, arom., J= 7.9), 7.75 (dd, 2H, arom., J= 8.0), 8.34 (d, 2H, arom., J= 1.7).
¹³C-NMR (CDCl₃) δ: 33.69, 110.87, 116.95, 129.84, 134.38, 134.73, 137.54, 145.58, 190.24.
MS: m/z 258 (M⁺, 9.41%)

### Example 2

Preparation of dibenzosuberone-3,7-dicarboxylic acid (4)

Suspension of 3,7-dicyano dibenzosuberone (3, 0.52 g, 2 mmola) in mixture of concentrated hydrochloric acid (25 ml) and acetic acid (25 ml) was stirred at reflux temperature during 34 h. Then, the crystals were separated by filtration, washed with water (2x10 ml) and dried. *Bis*-acid **4** (0.55 g) was obtained as pale brown powder, mp. > 330 °C (dec.).
IR (KBr) v: 2900, 2600, 1680, 1595, 1550, 1480, 1410, 1400, 1345, 1300, 1250, 1160, 1110, 1100, 1000, 930, 910, 875, 765, 750, 740, 630, 605.
¹H-NMR (DMSO-d₆) δ: 3.25 (s, 4H, benzyls), 7.49 (d, 2H, arom., J= 7.9), 8.10 (dd, 2H, arom., J= 7.8), 8.47 (d, 2H, arom., J= 1.5), 13.00 (COOH).
¹³C-NMR (DMSO-d₆) δ: 33.89, 129.62, 130.65, 131.67, 133.36, 137.88, 147.21, 166.94, 193.36.
Analysis for C₁₇H₁₀O₅ (296.28): Calc.: w(C)= 68.92%, w(H)= 4.08%, Found: w(C)= 68.98%, w(H)= 4.01%

### Example 3

Preparation of *N,N*'-*bis*-(3'dimethylamino-1'-propyl)dibenzosuberone-3,7-*bis*-carboxamide hydrochloride (**1**)

To the suspension of dibenzosuberone-3,7-dicarboxylic acid (4, 0.34 g, 1.15 mmol) in 10 ml of tetrahydrofuran was added thionylchloride (0.5 ml, 6.9 mmola) dropwise during 5 minutes. Reaction mixture was stirred at reflux temperature during 5 h. Then, the reaction mixture was allowed to cool to 0 °C and solution of 3-dimethylamino-1-propylamine (2.87 ml, 23 mmol) in THF (3 ml) was added dropwise at 0 °C. Reaction mixture was stirred at 0 °C during 1 hour and for 23 h at room temperature. Reaction mixture was evaporated and residue triturated with aqueous NaOH and extracted with 4x50 ml of dichloromethane. Organic layers were collected, dried over anhydrous sodium sulphate, filtered and evaporated. Solid product (0.40 g) was dissolved in 4 ml of absolute ethanol and dry hydrogenchloride was passed through the cooled solution Solution of the hydrochloride salt was evaporated and dried in high vacuum.
IR (KBr) ν: 3420, 2950, 2710, 1640, 1540, 1530, 1480, 1460, 1400, 1355, 1300, 1255, 1170, 1085, 1015, 845, 790, 760, 640, 600 cm⁻¹.
¹H-NMR (DMSO-d₆) δ: 1.97 (m, 4H, CH₂), 2.73 (s, 12H, N(CH₃)₂), 3.1 (m, 4H, CH₂), 3.23 (s, 4H, benzyls), 3.35 (m, 4H, CH₂), 7.47 (m, 2H, arom.), 8.07 (m, 2H, arom.), 8.39 (s, 2H, arom.), 8.98 (NH), 10.89 (NH⁺).
¹³C-NMR (DMSO-d₆) δ: 24.19, 33.77, 36.57, 41.95, 54.52, 129.44, 130.14, 131.45, 132.94, 137.90, 145.29, 165.73, 194.27.
MS: m/z 464 (M⁺, 52.94%)

## Claims

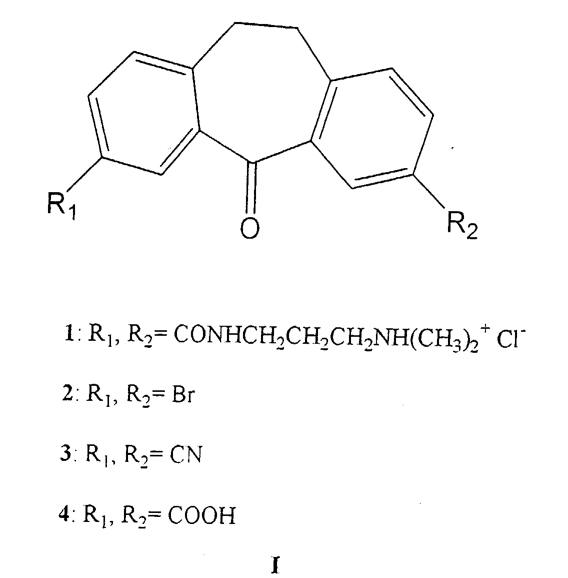

1. Dibenzosuberone derivative general formula **I**, **characterized by** the fact that the radicals R₁ and R₂ represent -CO-NHCH₂CH₂CH₂N(CH₃)₂ moeities as the free base or as *bis*-hydrochloride form.

2. Dibenzosuberone derivative general formula **I, characterized by** the fact that the radicals R₁ and R₂ represent CN (cyano) groups, compound **3.**

3. Preparation procedure for compound 1, pursuant to the Claim 1 general formula **I,** where radicals R₁ and R₂ represent -CO-NHCH₂CH₂CH₂N(CH₃)₂ moeities, **characterized** that compound 1 was obtained starting from 3,7-dibrom dibenzosuberone (2) by reaction with copper(I) cyanide in quinoline as reaction. solvent afforded 3,7-dicyano dibenzosuberone (3) which was hydrolysed with acid to gave diacid **4** followed by amidation with thionyl chloride and 3-dimethylamino-1-propylamine in lower ethers or chlorinated solvents. Base form of compound 1 was converted to his-hydrochloride salt by reaction with hydrogenchloride in lower aliphatic alcohols.
